# EUROPÄISCHE PATENTSCHRIFT

(11) **EP 1 390 365 B1**
(45) Veröffentlichungstag und Bekanntmachung des Hinweises auf die Patenterteilung: **02.02.2005**
(21) Anmeldenummer: 02745274.7
(22) Anmeldetag: 30.04.2002
(51) Int. Cl.: C07D 471/04, A61P 9/00, A61P 25/00

(54) **NEUE SULFONAT-SUBSTITUIERTE PYRAZOLOPYRIDINDERIVATE**
NOVEL SULFONATE SUBSTITUTED PYRAZOL PYRIDINE DERVIVATIVES
NOUVEAUX DERIVES PYRAZOLOPYRIDINE A SUBSTITUTION SULFONATE

(30) Priorität: 11.05.2001 DE 10122894
(43) Veröffentlichungstag der Anmeldung: 25.02.2004
(73) Patentinhaber: Bayer HealthCare AG, 51368 Leverkusen (DE)
(72) Erfinder: STASCH, Johannes-Peter, 42651 Solingen (DE); FEURER, Achim, 69259 Wilhelmsfeld (DE); WEIGAND, Stefan, 42115 Wuppertal (DE); STAHL, Elke, 51467 Bergisch Gladbach (DE); FLUBACHER, Dietmar, 79110 Freiburg (DE); ALONSO-ALIJA, Cristina, 42781 Haan (DE); WUNDER, Frank, 42117 Wuppertal (DE); LANG, Dieter, 42553 Velbert (DE); DEMBOWSKY, Klaus, 81475 München (DE); STRAUB, Alexander, 42117 Wuppertal (DE); PERZBORN, Elisabeth, 42327 Wuppertal (DE)
(86) Internationale Anmeldenummer: PCT/EP2002/004733
(87) Internationale Veröffentlichungsnummer: WO 2002/092596

(56) Entgegenhaltungen:
- DE-A- 10 057 751
- DE-A- 19 834 045
- DE-A- 19 834 047

## Beschreibung

Die vorliegende Erfindung betrifft neue chemische Verbindungen, welche die lösliche Guanylatcyclase stimulieren, ihre Herstellung und ihre Verwendung als Arzneimittel, insbesondere als Arzneimittel zur Behandlung von Herz-Kreislauf-Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriposphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch CO ist in der Lage, am Eisen-Zentralatom des Häms anzugreifen, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion und der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuifizienz, Thrombosen, Schläganfall und Myokardinfarkt führen kann.

Eine auf die Beeinflussung des cGMP-Signalweges in Organismen abzielende NOunabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriffe am Eisenzentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxymethyl-2'-furyl)-1-benzylindazol (YC-1, Wu et al., Blood 84 (1994), 4226; Mülsch et al., Br.J.Pharmacol. 120 (1997), 681), Fettsäuren (Goldberg et al, J. Biol. Chem. 252 (1977), 1279), Diphenyliodonium-hexafluorophosphat (Pettibone et al., Eur. J. Pharmacol. 116 (1985), 307), Isoliquiritigenin (Yu et al., Brit. J. Pharmacol. 114 (1995), 1587) sowie verschiedene substituierte Pyrazolderivate (WO 98/16223).

Weiterhin sind in der WO 98/16507, WO 98/23619, WO 00/06567, WO 00/06568, WO 00/06569 und WO 00/21954 Pyrazolopyridinderivate als Stimulatoren der löslichen Guanylatcyclase beschrieben. In diesen Patentanmeldungen sind auch Pyrazolopyridine beschrieben, welche einen Pyrimidinrest in 3-Position aufweisen. Derartige Verbindungen weisen eine sehr hohe in vitro Aktivität bezüglich der Stimulation der löslichen Guanylatcyclase auf Allerdings zeigte es sich, dass diese Verbindungen hinsichtlich ihrer in vivo-Eigenschaften wie beispielsweise ihrem Verhalten in der Leber, ihrem pharmakokinetischen Verhalten, ihrer Dosis-Wirkungsbeziehung oder ihrem Metabolisierungsweg einige Nachteile aufweisen.

Es war daher die Aufgabe der vorliegenden Erfindung, weitere Pyrazolopyridinderivate bereitzustellen, welche als Stimulatoren der löslichen Guanylatcyclase wirken, aber nicht die vorstehend aufgeführten Nachteile der Verbindungen aus dem Stand der Technik aufweisen.

Es war daher die Aufgabe der vorliegenden Erfindung, weitere Pyrazolopyridinderivate bereitzustellen, welche als Stimulatoren der löslichen Guanylatcyclase wirken, aber nicht die vorstehend aufgeführten Nachteile der Verbindungen aus dem Stand der Technik aufweisen.

Diese Aufgabe wird gemäß der vorliegenden Erfindungen durch Verbindungen gemäß Anspruch 1 gelöst. Diese neuen Pyrazolopyridinderivate zeichnen sich durch einen Pyrimidinrest in 3-Position aus, der ein bestimmtes Substitutionsmuster aufweist, nämlich einen Sulfonatrest in 5-Position des Pyrimidinrings sowie eine Aminogruppe in 4-Position des Pyrimidinrings.

Im einzelnen betrifft die vorliegende Erfindung Verbindungen der Formel (I) 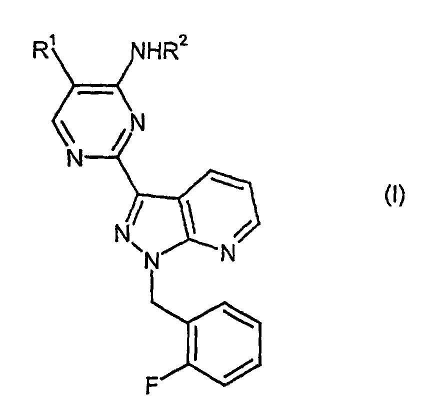 worin
- R¹: für einen Rest der Formel -O-SO₂-R³ steht,
wobei
R³ für einen Rest aus der Gruppe, bestehend aus gegebenenfalls substituiertem C₁₋₆-Alkyl, gegebenenfalls substituiertem C₃₋₈-Cycloalkyl, oder gegebenenfalls substituiertem Phenyl steht;
- R²: für H, gegebenenfalls substituiertes C₁₋₆-Alkyl-CO oder gegebenenfalls substituiertes C₁₋₆-Alkyl-SO₂- steht;
sowie Salze, Isomere und Hydrate davon.

Bevorzugt sind gemäß der vorliegenden Erfindung Verbindungen der Formel (I), bei denen
- R¹: für einen Rest der Formel -O-SO₂-R³ steht,
wobei
R³ für einen Rest aus der Gruppe, bestehend aus C₁₋₆-Alkyl, das gegebenenfalls mit einem bis drei Halogenresten substituiert ist, oder C₃₋₈-Cycloalkyl steht;
- R²: für H, gegebenenfalls mit einem bis drei Halogenresten substituiertes C₁₋₆-Alkyl-CO oder gegebenenfalls mit einem bis drei Halogenresten substituiertes C₁₋₆-Alkyl-SO₂- steht;
sowie Salze, Isomere und Hydrate davon.

Besonders bevorzugt sind hierbei Verbindungen der Formel (I), bei denen
- R¹: für einen Rest der Formel -O-SO₂-R³ steht,
wobei
R³ für einen Rest aus der Gruppe, bestehend aus Methyl, Ethyl, n-Propyl, iso-Propyl, n-Butyl, n-Pentyl, 1,1,1-Trifluor-4-n-butyl, Chlormethyl oder Cyclopropyl, steht;
- R²: für H oder CH₃CO steht;
sowie Salze, Isomere und Hydrate davon.

Die erfindungsgemäßen Verbindungen der allgemeinen Formel (I) können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, p-Toluolsulfonsäure; Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen.

Besonders bevorzugt sind z.B. Natrium-, Kalium-, Magnesium- oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren als auch deren jeweilige Mischungen. Die Racemformen lassen sich ebenso wie die Diastereomeren in bekannter Weise, beispielsweise durch chromatographische Trennung, in die stereoisomer einheitlichen Bestandteile trennen. In den erfindungsgemäßen Verbindungen vorhandene Doppelbindungen können in der cis- oder trans-Konfiguration (Z- oder E-Form) vorliegen.

Weiterhin können bestimmte Verbindungen in tautomeren Formen vorliegen. Dies ist dem Fachmann bekannt, und derartige Verbindungen sind ebenfalls vom Umfang der Erfindung umfasst.

Weiterhin können die erfindungsgemäßen Verbindungen in Form ihrer Hydrate vorkommen, wobei die Zahl der an das Molekül gebundenen Wassermoleküle von der jeweiligen erfindungsgemäßen Verbindung abhängt.

Im Rahmen der vorliegenden Erfindung haben die Substituenten soweit nicht anders angegeben im allgemeinen die folgende Bedeutung:
Alkyl steht im allgemeinen für einen geradkettigen oder verzweigten Kohlenwasserstoffrest mit 1 bis 6 Kohlenstoffatomen. Beispielsweise seien Methyl, Ethyl, Propyl, Isopropyl, Butyl, Isobutyl, Pentyl, Isopentyl, Hexyl, Isohexyl genannt.
Cycloalkyl steht im allgemeinen für einen cyclischen Kohlenwasserstoffrest mit 3 bis 8 Kohlenstoffatomen. Bevorzugt sind Cyclopropyl, Cyclopentyl und Cyclohexyl. Beispielsweise seien Cyclopentyl, Cyclohexyl, Cycloheptyl und Cyclooctyl genannt.
Halogen steht im Rahmen der Erfindung für Fluor, Chlor, Brom und Iod.

Die erfindungsgemäßen Verbindungen der Formel (I) können hergestellt werden durch
die Umsetzung der Verbindung der Formel (II) 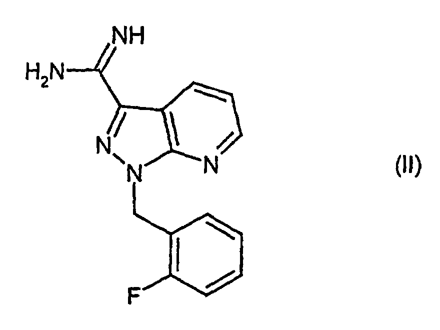 mit Verbindungen der Formel (III) 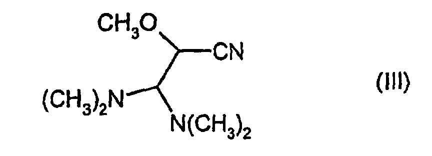 in einem organischen Lösungsmittel in Gegenwart einer Base unter Erhitzen und anschließender Überführung der Ethergruppe in die freie Hydroxygruppe zu Verbindungen der Formel (IV) 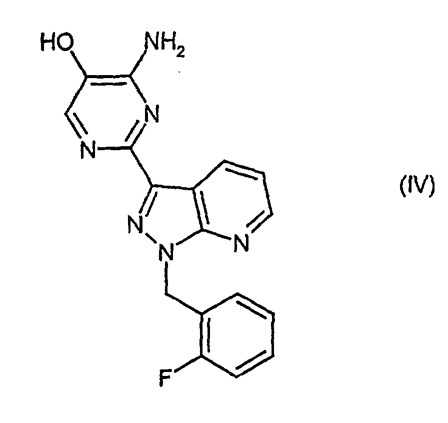 sowie die anschließende Umsetzung mit Verbindungen der Formel X-SO₂-R²
worin
- X: für eine durch eine Hydroxygruppe substituierbare Abgangsgruppe steht;
- R²: die vorstehend angegebene Bedeutung hat;
in einem organischen Lösungsmittel in Gegenwart einer Base unter Erhitzen zu Verbindungen der Formel (I).

Die Verbindung der Formel (II) lässt sich gemäß folgendem Reaktionsschema herstellen:

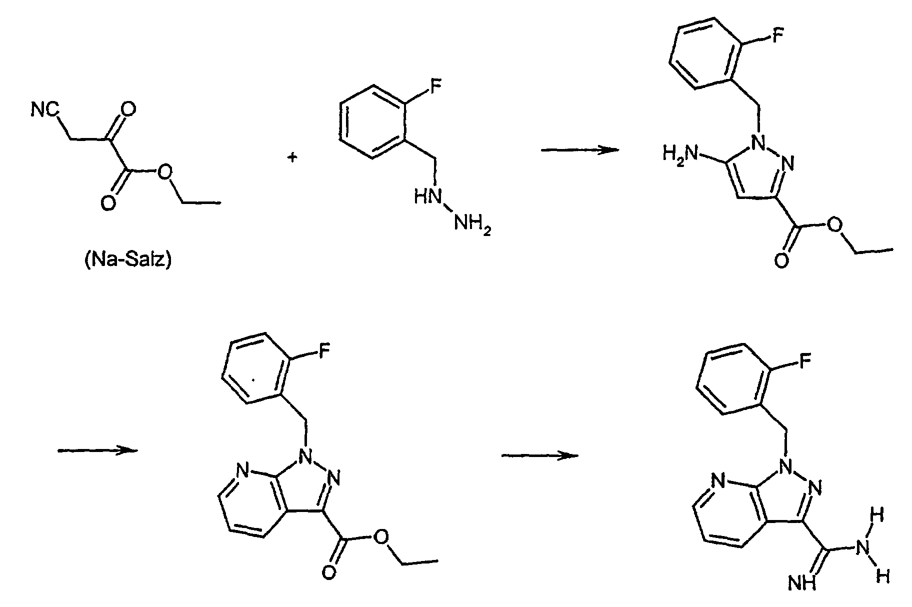

Die Verbindung der Formel (II) ist in einer mehrstufigen Synthese aus dem literaturbekannten Natriumsalz des Cyanobrenztraubensäureethylesters (Borsche und Manteuffel, Liebigs. Ann. Chem. 1934, 512, 97) erhältlich. Durch dessen Umsetzung mit 2-Fluorbenzylhydrazin unter Erhitzen und Schutzgasatmosphäre in einem inerten Lösungsmittel wie Dioxan erhält man den 5-Amino-1-(2-fluorbenzyl)-pyrazol-3-carbonsäureethylester, der durch Umsetzung mit Dimethylaminoacrolein im sauren Medium unter Schutzgasatmosphäre und Erhitzen zum entsprechenden Pyridinderivat cyclisiert. Dieses Pyridinderivat 1-(2-Fluorbenzyl)-1H-pyrazolo[3,4-b]pyridin-3-carbonsäureethylester wird durch eine mehrstufige Sequenz, bestehend aus Überführung des Esters mit Ammoniak in das entsprechende Amid, Dehydratisierung mit einem wasserentziehenden Mittel wie Trifluoressigsäureanhydrid zum entsprechenden Nitrilderivat, Umsetzung des Nitrilderivats mit Natriumethylat und abschließende Reaktion mit Ammoniumchlorid in die Verbindung der Formel (II) überführt.

Die Verbindung der Formel (III) kann aus den (z.B. bei Aldrich) käuflich erhältlichen Verbindungen t-Butoxybis(dimethylamino)methan und Methoxyacetonitril durch Umsetzung dieser Reaktanden vorzugsweise in äquimolaren Mengen vorzugsweise bei Normaldruck und Rühren der Reaktionslösung für mehrere Stunden, beispielsweise 12 Stunden, bei erhöhter Temperatur, beispielsweise 60-110°C, vorzugsweise 70-90°C, insbesondere 80°C hergestellt werden.

Die Umsetzung der Verbindungen der Formeln (II) und (III) zur Verbindung der Formel (IV) kann durch Einsatz der Reaktanden in äquimolaren Mengen beziehungsweise unter Verwendung der Verbindung der Formel (III) im leichten Überschuss in einem organischen Lösungsmittel, beispielsweise einem Alkohol, vorzugsweise Isoamylalkohol in Gegenwart einer geringen Menge einer Base, beispielsweise einem organischen Amin, insbesondere Piperidin, vorzugsweise bei Normaldruck und Rühren der Reaktionslösung für mehrere Stunden, beispielsweise 12 Stunden, bei erhöhter Temperatur, beispielsweise 60-130°C, vorzugsweise 80-120°C, insbesondere 110°C, und anschließende Freisetzung der Hydroxygruppe durch Umsetzung der so erhaltenen Verbindung mit einer vorzugsweise äquimolaren Menge eines Thiols wie beispielsweise Thiophenol in Gegenwart einer geringen Menge einer Base wie einer Alkalimetallbase, beispielsweise einem Alkalimetallcarbonat, vorzugsweise Kaliumcarbonat in einem organischen Lösungsmittel wie beispielsweise 1-Methyl-2-pyrrolidon vorzugsweise bei Normaldruck und Rühren der Reaktionslösung für mehrere Stunden, beispielsweise 1 Stunde, bei erhöhter Temperatur, beispielsweise 100-200°C, vorzugsweise 150-200°C, durchgeführt werden.

Die so erhaltene Verbindung der Formel (IV) kann durch Umsetzung mit einer äquimolaren Menge oder eines leichten Überschusses einer Sulfonylverbindung der Formel XSO₂R₂ in die erfindungsgemäßen Verbindungen der Formel (I) überführt werden. Die Reaktion wird in Gegenwart einer geringen Menge einer Base wie einem organischen Amin, vorzugsweise Pyridin vorzugsweise bei Normaldruck und Rühren der Reaktionslösung für mehrere Stunden, beispielsweise 12 Stunden, bei erhöhter Temperatur, beispielsweise 40-80°C, vorzugsweise 50-70°C durchgeführt. Die Sulfonylverbindungen sind käuflich erhältlich oder auf dem Fachmann bekannte Weise zugänglich.

Die erfindungsgemäßen Verbindungen der allgemeinen Formel (I) zeigen ein nicht vorhersehbares, wertvolles pharmakologisches Wirkspektrum.

Die erfindungsgemäßen Verbindungen der allgemeinen Formel (I) führen zu einer Gefäßrelaxation, Thrombozytenaggregationshemmung und zu einer Blutdrucksenkung sowie zu einer Steigerung des koronaren Blutflusses. Diese Wirkungen sind über eine direkte Stimulation der löslichen Guanylatzyklase und einem intrazellulären cGMP-Anstieg vermittelt. Außerdem verstärken die erfindungsgemäßen Verbindungen der allgemeinen Formel (I) die Wirkung von Substanzen, die den cGMP-Spiegel steigern, wie beispielsweise EDRF (Endothelium derived relaxing factor), NO-Donatoren, Protoporphyrin IX, Arachidonsäure oder Phenylhydrazinderivate.

Sie können daher in Arzneimitteln zur Behandlung von kardiovaskulären Erkrankungen wie beispielsweise zur Behandlung des Bluthochdrucks und der Herzinsuffizienz, stabiler und instabiler Angina pectoris, peripheren und kardialen Gefäßerkrankungen, von Arrhythmien, zur Behandlung von thromboembolischen Erkrankungen und Ischämien wie Myokardinfarkt, Himschlag, transistorisch und ischämische Attacken, periphere Durchblutungsstörungen, Verhinderung von Restenosen wie nach Thrombolysetherapien, percutan transluminalen Angioplastien (PTA), percutan transluminalen Koronarangioplastien (PTCA), Bypass sowie zur Behandlung von Arteriosklerose, asthmatischen Erkrankungen und Krankheiten des Urogenitalsystems wie beispielsweise Prostatahypertrophie, erektile Dysfunktion, weibliche sexuelle Dysfunktion, Osteoporose, Gastroparese und Inkontinenz eingesetzt werden.

Die in der vorliegenden Erfindung beschriebenen Verbindungen der allgemeinen Formel (I) stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung, oder Gedächtnisleistung nach kognitiven Störungen, wie sie insbesondere bei Situationen/Krankheiten/Syndromen auftreten wie "Mild cognitive impairment", Altersassoziierte Lern- und Gedächtnisstörungen, Altersassoziierte Gedächtnisverluste, Vaskuläre Demenz, Schädel-Hirn-Trauma, Schlaganfall, Demenz, die nach Schlaganfällen auftritt ("post stroke dementia"), post-traumatisches Schädel Hirn Trauma, allgemeine Konzentrationsstörungen, Konzentrationsstörungen in Kindern mit Lern- und Gedächtnisproblemen, Alzheimersche Krankheit, Vaskuläre Demenz, Demenz mit Lewy-Körperchen, Demenz mit Degeneration der Frontallappen einschließlich des Pick's Syndroms, Parkinsonsche Krankheit, Progressive nuclear palsy, Demenz mit corticobasaler Degeneration, Amyolateralsklerose (ALS), Huntingtonsche Krankheit, Multiple Sklerose, Thalamische Degeneration, Creutzfeld-Jacob-Demenz, HIV-Demenz, Schizophrenie mit Demenz oder Korsakoff-Psychose. Sie eignen sich auch zur Behandlung von Erkrankungen des Zentralnervensystems wie Angst-, Spannungs- und Depressionszuständen, zentralnervös bedingten Sexualdysfunktionen und Schlafstörungen, sowie zur Regulierung krankhafter Störungen derNahrungs-, Genuss- und Suchtmittelaufnahme.

Weiterhin eignen sich die Wirkstoffe auch zur Regulation der cerebralen Durchblutung und stellen somit wirkungsvolle Mittel zur Bekämpfung von Migräne dar.

Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen der allgemeinen Formel (I) zur Bekämpfung von Schmerzzuständen eingesetzt werden.

Zudem besitzen die erfindungsgemäßen Verbindungen antiinflammatorische Wirkung und können daher als entzündungshemmende Mittel eingesetzt werden.

Darüber hinaus umfasst die Erfindung die Kombination der erfindungsgemäßen Verbindungen der allgemeinen Formel (I) mit organischen Nitraten und NO-Donatoren.

Organische Nitrate und NO-Donatoren im Rahmen der Erfindung sind im allgemeinen Substanzen, die über die Freisetzung von NO bzw. NO-Species ihre therapeutische Wirkung entfalten. Bevorzugt sind Natriumnitroprussid, Nitroglycerin, Isosorbiddinitrat, Isosorbidmononitrat, Molsidomin und SIN-1.

Außerdem umfasst die Erfindung die Kombination mit Verbindungen, die den Abbau von cyclischem Guanosinmonophosphat (cGMP) inhibieren. Dies sind insbesondere Inhibitoren der Phosphodiesterasen 1, 2 und 5; Nomenklatur nach Beavo und Reifsnyder (1990) TiPS 11 S. 150 bis 155. Durch diese Inhibitoren wird die Wirkung der erfindungsgemäßen Verbindung potenziert und der gewünschte pharmakologische Effekt gesteigert.

### Biologische Untersuchungen

### Gefäßrelaxierende Wirkung in vitro

Kaninchen werden durch Nackenschlag betäubt und entblutet. Die Aorta wird entnommen, von anhaftendem Gewebe befreit, in 1,5 mm breite Ringe geteilt und einzeln unter einer Vorspannung in 5 ml-Organbäder mit 37°C warmer, carbogenbegaster Krebs-Henseleit-Lösung folgender Zusammensetzung (mM) gebracht: NaCl: 119; KCl: 4,8; CaCl₂ x 2 H₂O: 1; MgSO₄ x 7 H₂O; 1,4; KH₂PO₄: 1,2; NaHCO₃:25; Glucose: 10. Die Kontraktionskraft wird mit Statham UC2-Zellen erfasst, verstärkt und über A/D-Wandler (DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt. Nach mehreren Kontrollzyklen wird die zu untersuchende Substanz in jedem weiteren Durchgang in jeweils steigender Dosierung untersucht und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50 % zu reduzieren (IC₅₀). Das Standardapplikationsvolumen beträgt 5 µl, der DMSO-Anteil in der Badlösung entspricht 0,1 %. Die Ergebnisse sind nachstehend in Tabelle 1 aufgeführt:

**Tabelle 1:**

| **Gefäßrelaxierende Wirkung in vitro** | |
|---|---|
| Beispiel Nr. | IC₅₀ [nM] |
| 1 | 700 |
| 2 | 580 |
| 3 | 300 |
| 4 | 710 |
| 5 | 520 |
| 7 | 440 |
| 10 | 2020 |

### Bestimmung der Leberclearance in vitro

Ratten werden anästhesiert, heparinisiert, und die Leber in situ über die Pfortader perfundiert. Ex vivo werden dann aus der Leber mittels Collagenase-Lösung die primären Ratten-Hepatozyten gewonnen. Es wurden 2·10⁶ Hepatozyten pro ml mit jeweils der gleichen Konzentration der zu untersuchenden Verbindung bei 37°C inkubiert. Die Abnahme des zu untersuchenden Substrates über die Zeit wurde bioanalytisch (HPLC/UV, HPLC/Fluoreszenz oder LC/MSMS) an jeweils 5 Zeitpunkten im Zeitraum von 0-15 min nach Inkubationsstart bestimmt. Daraus wurde über Zellzahl und Lebergewicht die Clearance errechnet.

### Bestimmung der Plasmaclearance in vivo

Die zu untersuchende Substanz wird Ratten über die Schwanzvene intravenös als Lösung appliziert. Zu festgelegten Zeitpunkten wird den Ratten Blut entnommen, dieses wird heparinisiert und durch herkömmliche Maßnahmen Plasma daraus gewonnen. Die Substanz wird im Plasma bioanalytisch quantifiziert. Aus den so ermittelten Plasmakonzentrations-Zeit-Verläufen werden über herkömmliche hierfür verwendete nicht-kompartimentelle Methoden die pharmakokinetischen Parameter errechnet.

Zur vorliegenden Erfindung gehören pharmazeutische Zubereitungen, die neben nichttoxischen, inerten pharmazeutisch geeigneten Trägerstoffen die erfindungsgemäßen Verbindungen der allgemeinen Formel (I) enthält sowie Verfahren zur Herstellung dieser Zubereitungen.

Die Wirkstoff können gegebenenfalls in einem oder mehreren der oben angegebenen Trägerstoffe auch in mikroverkapselter Form vorliegen.

Die therapeutisch wirksamen Verbindungen der allgemeinen Formel (I) sollen in den oben aufgeführten pharmazeutischen Zubereitungen in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen der allgemeinen Formel (I) auch weitere pharmazeutische Wirkstoffe enthalten.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,01 bis etwa 700, vorzugsweise 0,01 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 0,1 bis etwa 80, insbesondere 0,1 bis 30 mg/kg Körpergewicht.

Die vorliegende Erfindung wird nachstehend anhand von nicht einschränkenden bevorzugten Beispielen näher dargestellt. Soweit nicht anderweitig angegeben, beziehen sich alle Mengenangaben auf Gewichtsprozente.

### Beispiele

### Abkürzungen:

- RT:: Raumtemperatur
- EE:: Essigsäureethylester
- MCPBA:: m-Chlorperoxybenzoesäure
- BABA:: n-Butylacetat/n-Butanol/Eisessig/Phosphatpuffer pH 6 (50:9:25.15; org. Phase)
- DMF:: N,N-Dimethylformamid

### Laufmittel für die Dünnschichtchromatographie:

- T1 E1:: Toluol - Essigsäureethylester (1:1)
- T1 EtOH1:: Toluol-Methanol (1:1)
- C1 E1:: Cyclohexan - Essigsäureethylester (1: 1)
- C1 E2:: Cyclohexan - Essigsäureethylester (1:2)

### Methoden zur Ermittlung der HPLC-Retentionszeiten:

### Methode A (HPLC-MS):

| | |
|---|---|
| Eluent | A= CH₃CN B= 0.6 g 30 %ige HCl/l H₂O |
| Fluss | 0.6 ml/min |
| Säulenofen | 50°C |
| Säule | Symmetry C18 2.1*150mm |

| Gradient: | | | |
|---|---|---|---|
| Zeit (min) | %A | %B | Fluss (ml/min) |
| 0 | 10 | 90 | 0.6 |
| 4 | 90 | 10 | 0.6 |
| 9 | 90 | 10 | 0.8 |

### Methode B (HPLC):

| | |
|---|---|
| Eluent | A=5 ml HClO₄/l H₂O, B=CH₃CN |
| Fluss | 0.75 ml/min |
| L-R Temperatur | 30.00°C 29.99°C |
| Säule | Kromasil C18 60*2mm |

| Gradient: | | |
|---|---|---|
| Zeit (min) | %A | %B |
| 0.50 | 98 | 2 |
| 4.50 | 10 | 90 |
| 6.50 | 10 | 90 |
| 6.70 | 98 | 2 |
| 7.50 | 98 | 2 |

### Methode C (HPLC):

| | |
|---|---|
| Eluent | A= H₃PO₄ 0.01 mol/l, B=CH₃CN |
| Fluss | 0.75 ml/min |
| L-R Temperatur | 30.01°C 29.98°C |
| Säule | Kromasil C18 60*2mm |

| Gradient: | | |
|---|---|---|
| Zeit (min) | %A | %B |
| 0.00 | 90 | 10 |
| 0.50 | 90 | 10 |
| 4.50 | 10 | 90 |
| 8.00 | 10 | 90 |
| 8.50 | 90 | 10 |
| 10.00 | 90 | 10 |

### Methode D (chirale HPLC):

| | |
|---|---|
| Eluent | 50 % iso-Hexan, 50 % Ethanol |
| Fluss | 1.00 ml/min |
| Temperatur | 40°C |
| Säule | 250*4,6 mm, gefüllt mit Chiralcel OD, 10 µm |

### Methode E (HPLC-MS):

| | |
|---|---|
| Eluent | A= CH₃CN B= 0.3 g 30 %ige HCl /l H₂O |
| Fluss | 0.9 ml/min |
| Säulenofen | 50°C |
| Säule | Symmetry C18 2.1*150mm |

| Gradient: | | | |
|---|---|---|---|
| Zeit (min) | %A | %B | Fluss (ml/min) |
| 0 | 10 | 90 | 0.9 |
| 3 | 90 | 10 | 1.2 |
| 6 | 90 | 10 | 1.2 |

### Ausgangsverbindungen:

### I. Synthese von 3,3-Bis(dimethylamino)-2-methoxypropionitril

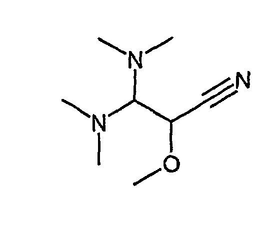

40.0 g (229.5 mmol) ter-Butoxybis(dimethylamino)methan und 16.3 g (229.5 mmol) Methoxyacetonitril werden über Nacht bei 80°C gerührt. Zur Aufarbeitung wird flüchtiges Material am Rotationsverdampfer abgezogen und der Rückstand im Kugelrohr bei 140°C im Hochvakuum destilliert. Das Produkt enthält laut NMR-Spektrum (300 MHz, D₆-DMSO) das Enamin als E/Z-Gemisch, das durch Eliminierung von Dimethylamin entsteht. Die Produktmischung wird ohne weitere Reinigung in die nächste Reaktion eingesetzt.
Ausbeute: 24.7 g (60 %)

### II. Synthese von 1-(2-Fluorbenzyl)1H-pyrazolo[3,4-b]pyridin-3-carboxamidin

### 2A) 5-Amino-1-(2-fluorbenzyl)-pyrazol-3-carbonsäureethylester

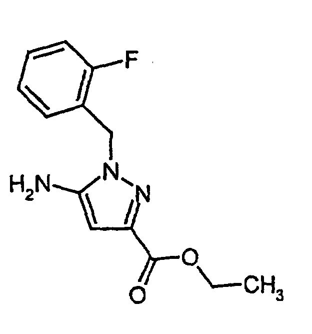

100 g (0.613 mol) Natriumsalz des Cyanobrenztraubensäureethylester (Darstellung analog Borsche und Manteuffel, Liebigs Ann. 1934, *512*, 97) werden unter gutem Rühren unter Argon in 2.5 1 Dioxan bei Raumtemperatur mit 111.75 g (75 ml, 0.98 mol) Trifluoressigsäure versetzt und 10 min gerührt, wobei ein großer Teil des Eduktes in Lösung geht. Dann gibt man 85.93 g (0.613 mol) 2-Fluorbenzylhydrazin hinzu und kocht über Nacht. Nach Abkühlen werden die ausgefallenen Kristalle des Natriumtrifluoracetats abgesaugt, mit Dioxan gewaschen und die Lösung roh weiter umgesetzt.

### 2B) 1-(2-Fluorbenzyl)-1H-pyrazolo[3,4-b]pyridin-3-carbonsäureethylester

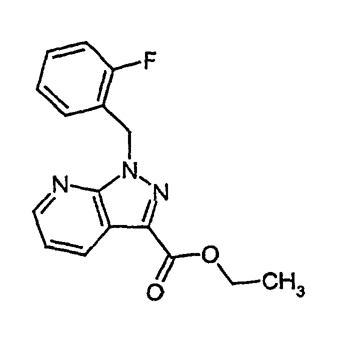

Die aus 2A) erhaltene Lösung wird mit 61.25 ml (60.77 g, 0.613 mol) Dimethylaminoacrolein und 56.28 ml (83.88 g, 0.736 mol) Trifluoressigsäure versetzt und unter Argon 3 Tage lang gekocht. Anschließend wird das Lösungsmittel im Vakuum verdampft, der Rückstand in 2 l Wasser gegeben und dreimal mit je 1 l Essigester extrahiert. Die vereinigten organischen Phasen werden mit Magnesiumsulfat getrocknet und einrotiert. Man chromatographiert auf 2.5 kg Kieselgel und eluiert mit einem Toluol/Toluol-Essigester = 4:1-Gradienten. Ausbeute: 91.6 g (49.9 % d.Th. über zwei Stufen).
Smp. 85°C
R_{f}(SiO₂, T1E1): 0.83

### 2C) 1-(2-Fluorbenzyl)-1H-pyrazolo[3,4-b]pyridin-3-carboxamid

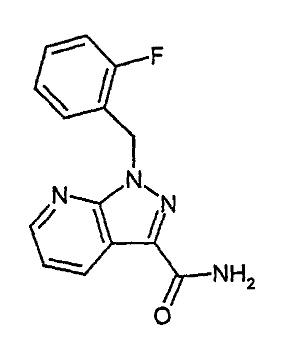

10.18 g (34 mmol) des in Beispiel 2B) erhaltenen Esters werden in 150 ml mit Ammoniak bei 0 - 10°C gesättigtem Methanol vorgelegt. Man rührt zwei Tage bei Raumtemperatur und engt anschließend im Vakuum ein.
R_{f} (SiO₂, T1E1): 0.33

### 2D) 3-Cyano-1-(2-fluorbenzyl)-1H-pyrazolo[3,4-b]pyridin

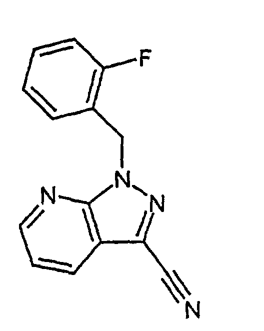

36.1 g (133 mmol) 1-(2-Fluorbenzyl)-1H-pyrazolo[3,4-b]pyridin-3-carboxamid aus Beispiel 2C) werden in 330 ml THF gelöst und mit 27 g (341 mmol) Pyridin versetzt. Anschließend gibt man innerhalb von 10 min 47.76 ml (71.66 g, 341 mmol) Trifluoressigsäureanhydrid hinzu, wobei die Temperatur bis auf 40°C ansteigt. Man rührt über Nacht bei Raumtemperatur. Anschließend wird der Ansatz in 1 l Wasser gegeben und dreimal mit je 0.5 l Essigester extrahiert. Die organische Phase wird mit gesättigter Natriumhydrogencarbonatlösung und mit 1 N HCl gewaschen, mit MgSO4 getrocknet und einrotiert.
Ausbeute: 33.7 g (100 % d.Th.)
Smp: 81°C
R_{f} (SiO₂, T1E1): 0.74

### 2E) (2-Fluorbenzyl)-1H-pyrazolo[3,4-b]pyridin-3-carboximidsäuremethylester

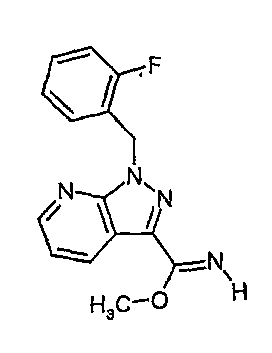

Man löst 30.37 g (562 mmol) Natriummethylat in 1.5 l Methanol und gibt 36.45 g (144.5 mmol) 3-Cyano-1-(2-fluorbenzyl)-1H-pyrazolo[3,4-b]pyridin (aus Beispiel 2D) hinzu. Man rührt 2 Stunden bei Raumtemperatur und setzt die erhaltene Lösung direkt für die nächste Stufe ein.

### 2F) 1-(2-Fluorbenzyl)1H-pyrazolo[3,4-b]pyridin-3-carboxamidin

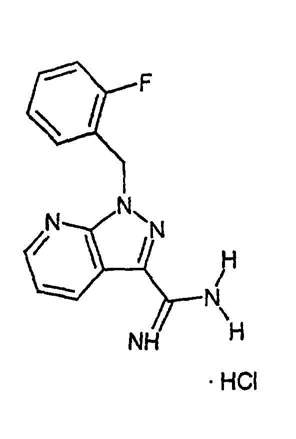

Die aus Beispiel 2E) erhaltene Lösung von (2-Fluorbenzyl)-1H-pyrazolo[3,4-b]-pyridin-3-carboximidsäuremethylester in Methanol wird mit 33.76 g (32.19 ml, 562 mmol) Eisessig und 9.28 g (173 mmol) Ammoniumchlorid versetzt und über Nacht unter Rückfluss gerührt. Man verdampft das Lösungsmittel im Vakuum, verreibt den Rückstand gut mit Aceton und saugt den ausgefallenen Feststoff ab.
¹H-NMR (d₆-DMSO, 200 MHz): δ= 5,93 (s, 2H); 7,1-7,5 (m, 4 H); 7,55 (dd, 1H); 8,12 (dd, 1H); 8,30 (dd, 1H); 9,5 (bs, 4H-austauschbar) ppm.
MS (EI): m/z = 270,2 (M-HCl)

### III. Synthese von 2-[1-(2-Fluorobenzyl)-1H-pyrazolo[3,4-b]pyridin-3-yl]-5-methoxy-4-pyrimidinylamin

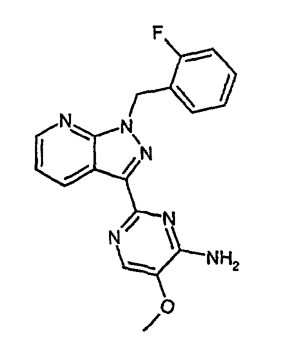

46.8 g (134.8 mmol) 1-(2-Fluorobenzyl)-1H-pyrazolo[3,4-b]pyridin-3-carboximidamid aus Beispiel II werden in Isoamylalkohol gelöst. Dazu gibt man 24.7 g (144.2 mmol) 3,3-bis(dimethylamino)-2-methoxypropionitril aus Beispiel I und 1.15 g (1.33 ml, 13.5 mmol) Piperidin und lässt 3 Tage bei 110°C rühren. Zur Aufarbeitung kühlt man auf 0°C, saugt das ausgefallene Produkt ab, wäscht gut mit kaltem Diethylether und trocknet im Vakuumtrockenschrank bei 50°C.
Ausbeute: 25.4 g (52.7 %) .
R_{f}-Wert: 0.34 (Dichlormethan/Methanol 20:1)
¹H-NMR: (400 MHz, D₆-DMSO), δ = 3.89 (2, 3H, OCH₃), 5.79 (s, 2H, CH₂), 6.93 (br. s, 2H, NH₂), 7.10-7.26 (m, 3H, Ar-H), 7.31-7.39 (m, 2H, Ar-H),
7.98 (s, 1H, Pyrirnidin-H), 8.61 (dd, 1H, Pyridin-H), 8.92 (dd, 1H, Pyridin-H)
MS: (ESI pos.), *m*/*z* = 350.9 ([M+H]⁺), 700.8 ([2M+H]⁺)

### IV. Synthese von 4-Amino-2-[1-(2-fluorobenzyl)-1H-pyrazolo[3,4-b]pyridin-3-yl]-5-pyrimidinol

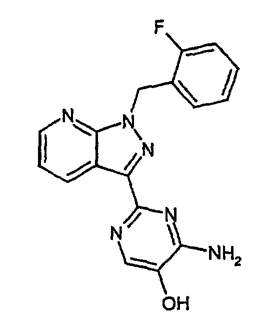

25.3 g (72.2 mmol) 2-[1-(2-fluorobenzyl)-1H-pyrazolo[3,4-b]pyridin-3-yl]-5-methoxy-4-pyrimidinylamin aus Beispiel III werden in 500 ml 1-Methyl-2-pyrrolidon gelöst. Dazu gibt man 7.96 g (7.42 ml, 72.2 mmol) Thiophenol und 2.50 g (18.1 mmol) Kaliumcarbonat und lässt ca. 1h bei 190°C rühren. Zur Aufarbeitung wird das Lösungsmittel abkondensiert, der Rückstand mit halbkonz. Ammoniumchlorid-Lösung versetzt und dreimal mit Ethylacetat extrahiert. Dabei fällt das Produkt größtenteils aus. Es wird abgesaugt und im Vakuumtrockenschrank bei 50°C getrocknet.
- Ausbeute:: 18.1 g (72.3 %)
- R_{f}-Wert:: 0.44 (Dichlormethan/Methanol 10:1)
- ¹H-NMR:: (300 MHz, D₆-DMSO), δ = 5.78 (s, 2H, CH₂), 6.66 (br. s, 2H, NH₂), 7.09-7.38 (m, 5H, Ar-H), 7.82 (s, 1H, Pyrimidin-H), 8.60 (dd, 1H, Pyridin-H), 8.92 (dd, 1H, Pyridin-H), 9.4-10.2 (br. s, 1H, OH)
- MS:: (ESI pos.), *m*/*z* = 337.3 ([M+H]⁺), 673.3 ([2M+H]⁺)

### Beispiele

### 1. 4-Amino-2-[1-(2-fluorobenzyl)-1H-pyrazolo[3,4-b]pyridin-3-yl]-5-pyrimidinyl-chloromethansulfonat

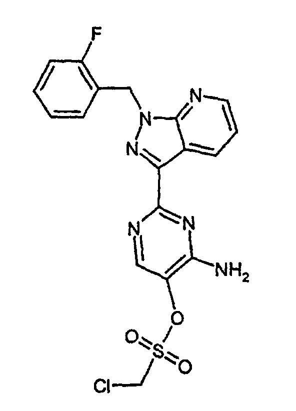

400 mg (1.19 mmol) 4-amino-2-[1-(2-fluorobenzyl)-1H-pyrazolo[3,4-b]pyridin-3-yl]-5-pyrimidinol aus Beispiel IV wurden in 8.0 ml Pyridin suspendiert und mit 186.1 mg (1.25 mmol) Chlormethansulfonylchlorid versetzt. Die Suspension wurde bei 60°C über Nacht gerührt und anschließend das Gemisch mit Wasser versetzt. Der entstandene Niederschlag wurde abgesaugt, mehrmals mit Wasser gewaschen und im Hochvakuum getrocknet.
- Ausbeute:: 480 mg (77.3 %)
- ¹H-NMR:: (400 MHz, D₆-DMSO), δ = 5.76 (s, 2H, CH₂), 5.82 (s, 2H, CH₂), 6.66 (br. s, 2H, NH₂), 7.10-7.26 (m, 3H, Ar-H), 7.30-7.42 (m, 2H, Ar-H),
7.59 (br. s, 2H, NH₂), 8.31 (s, 1H, Pyrimidin-H), 8.65 (dd, 1H, Pyridin-H),
8.93 (dd, 1H, Pyridin-H)
- MS:: (ESI pos.), *m*/*z* = 449 ([M+H]⁺), 897 ([2M+H]⁺)

Auf analoge Weise wurden hergestellt:

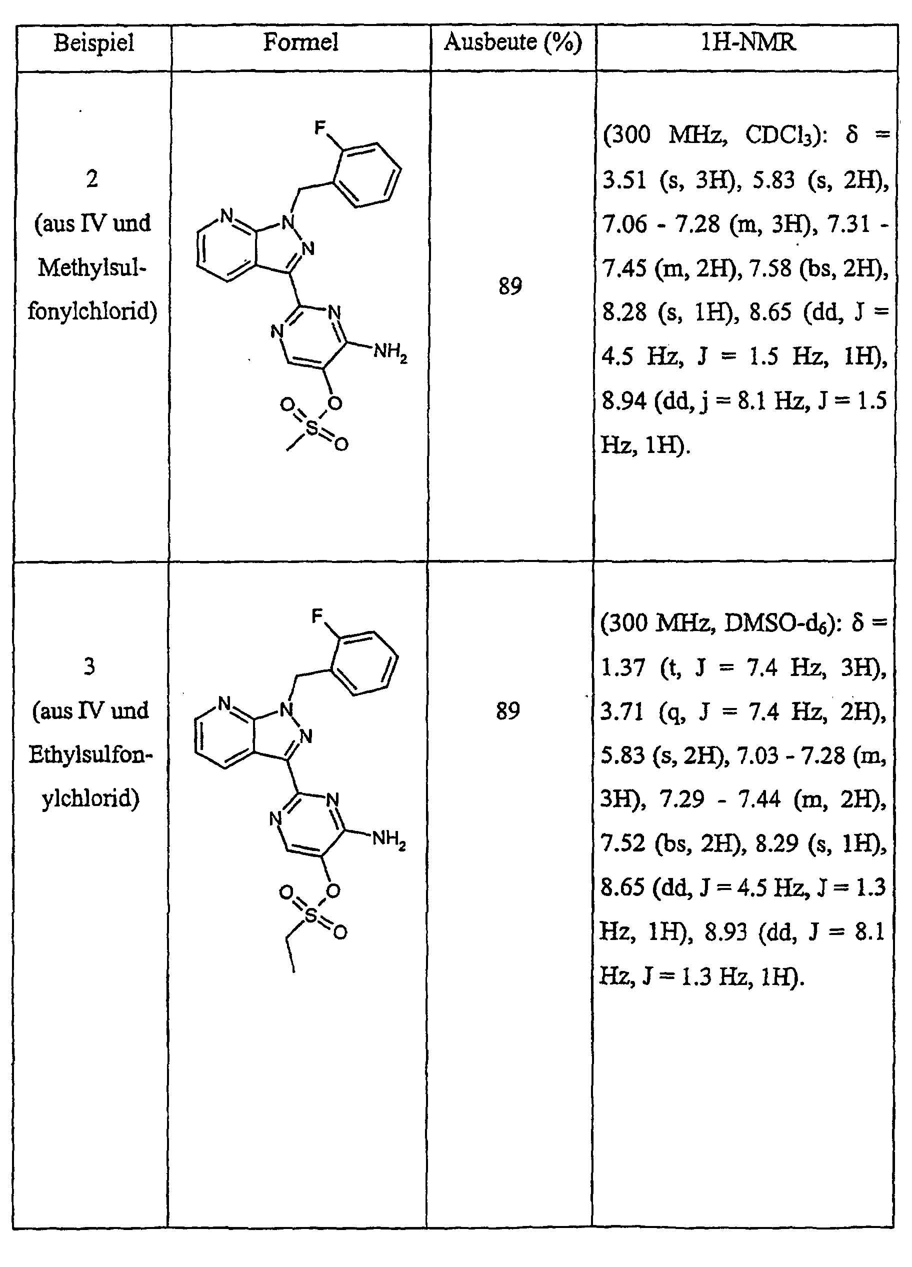

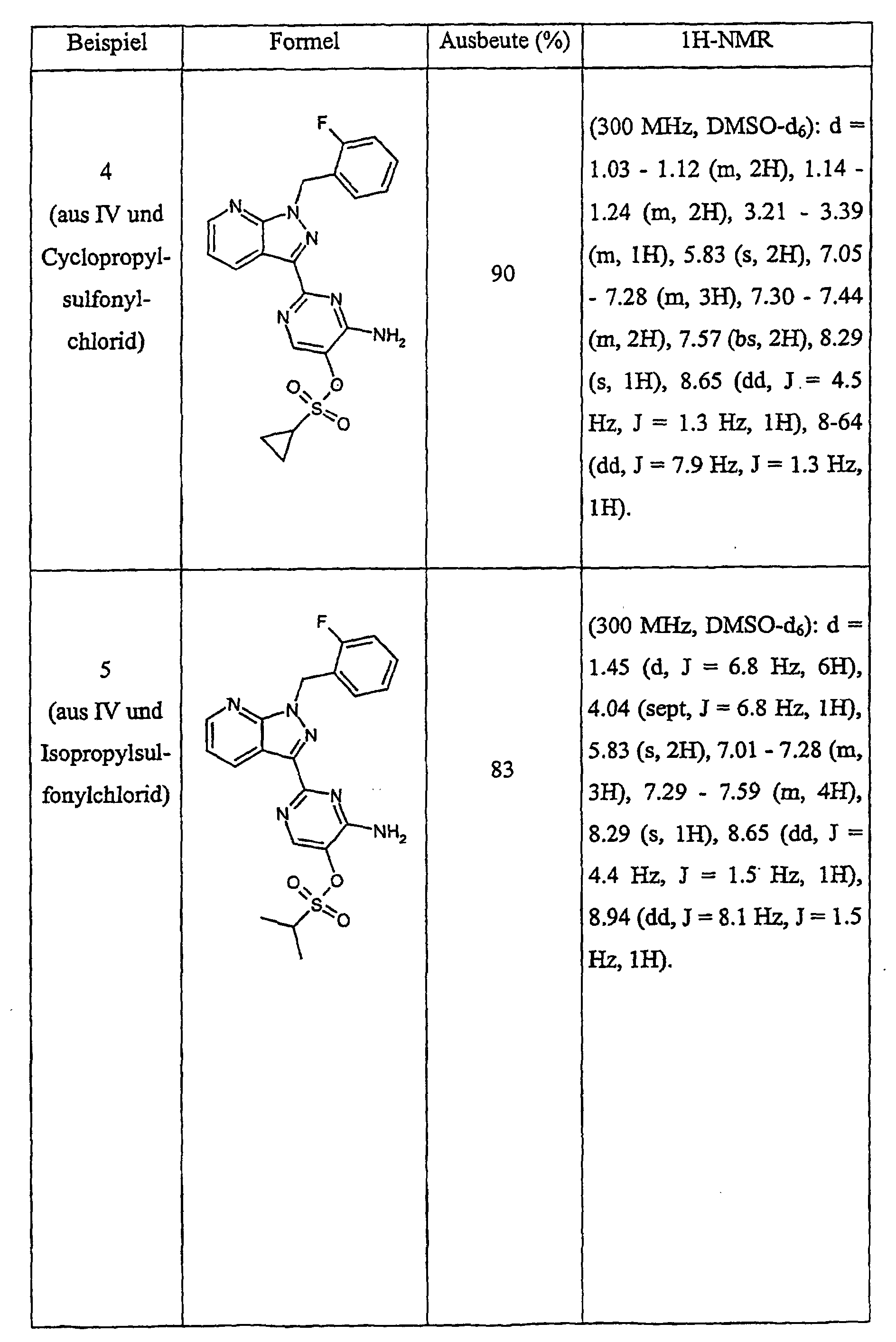

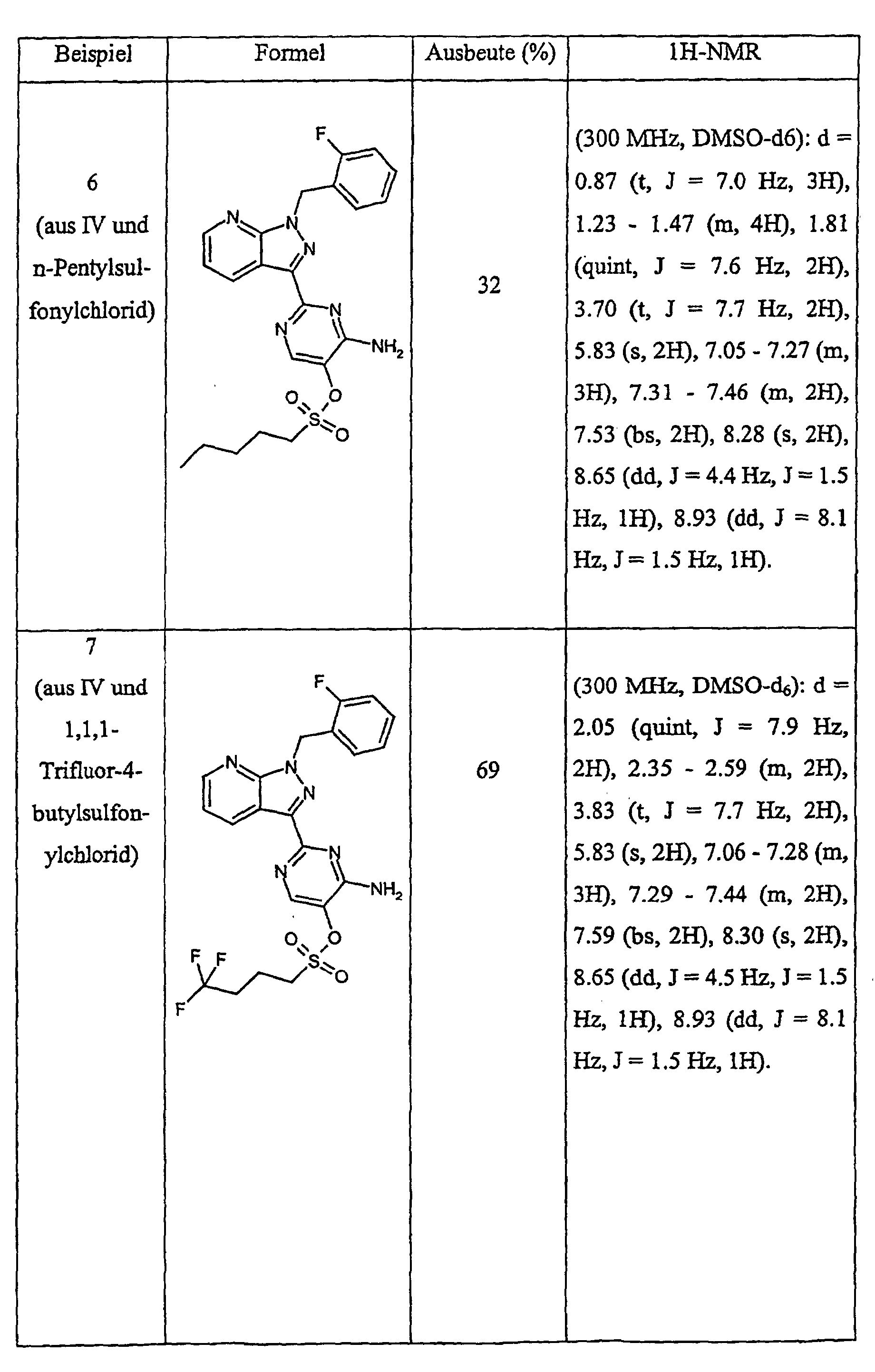

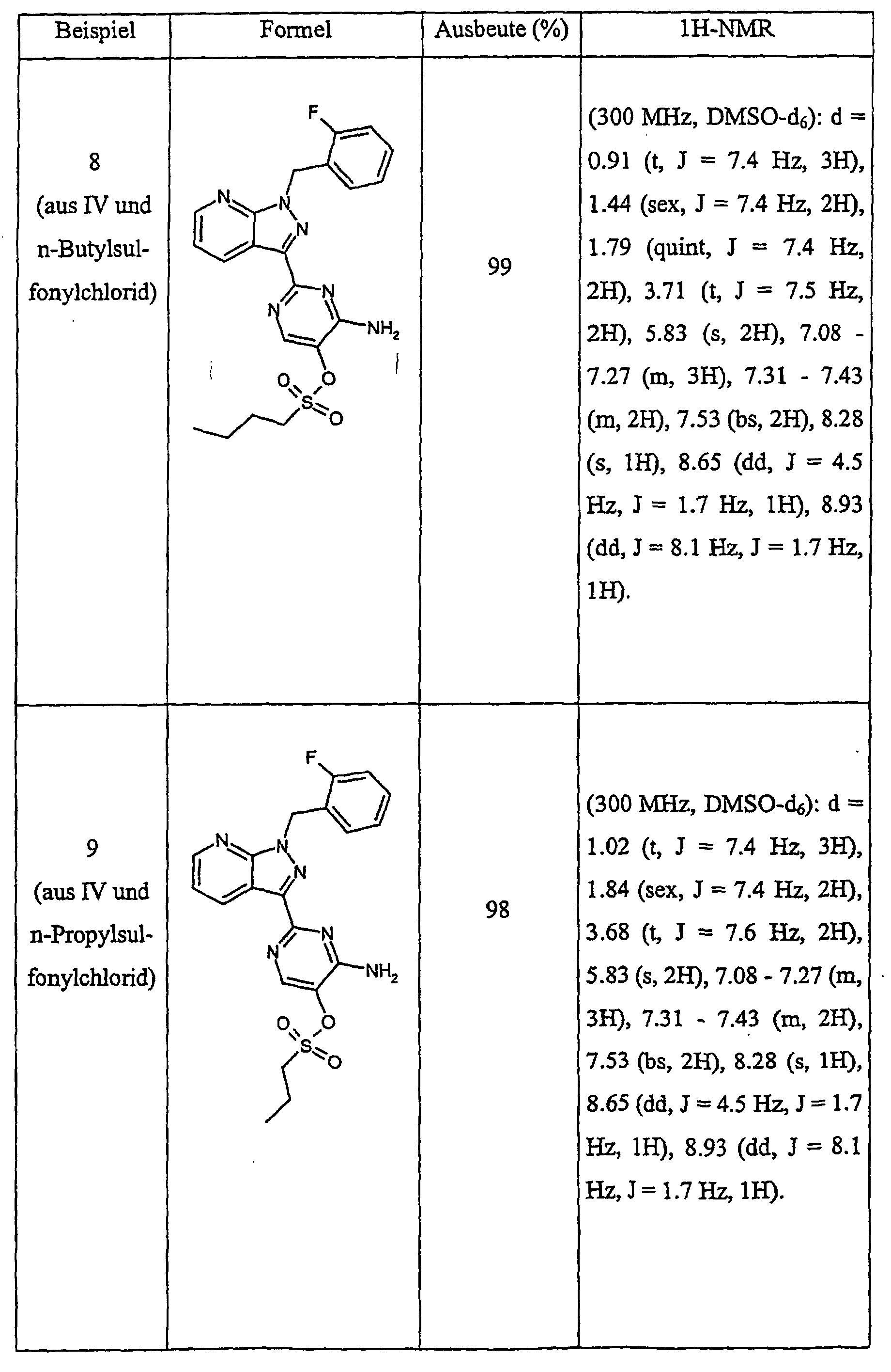

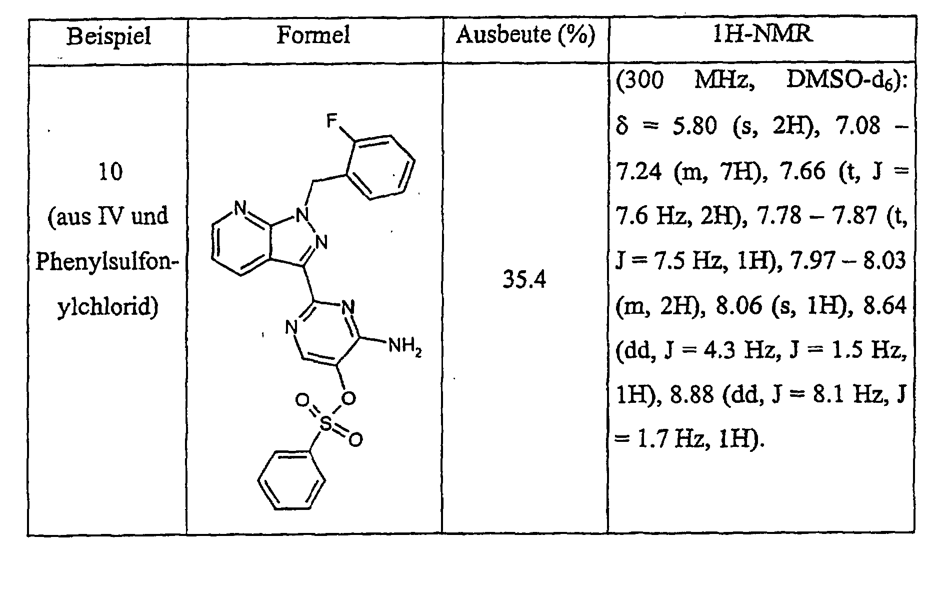

## Patentansprüche

1. Verbindungen der allgemeinen Formel (I) 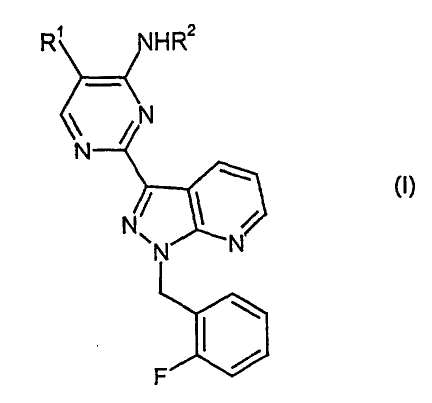 worin
R¹ für einen Rest der Formel -O-SO₂-R³ steht,
wobei
R³ für einen Rest aus der Gruppe, bestehend aus gegebenenfalls substituiertem C₁₋₆-Alkyl, gegebenenfalls substituiertem C₃₋₈-Cycloalkyl, oder gegebenenfalls substituiertem Phenyl steht;
R² für H, gegebenenfalls substituiertes C₁₋₆-Alkyl-CO oder gegebenen-falls substituiertes C₁₋₆-Alkyl-SO₂- steht;
sowie Salze, Isomere und Hydrate davon.

2. Verbindungen nach Anspruch 1,
worin
R¹ für einen Rest der Formel -O-SO₂-R³ steht,
wobei
R³ für einen Rest aus der Gruppe, bestehend aus C₁₋₆-Alkyl, das gegebenenfalls mit einem bis drei Halogenresten substituiert ist, oder C₃₋₈-Cycloalkyl steht;
R² für H, gegebenenfalls mit einem bis drei Halogenresten substituiertes C₁₋₆-Alkyl-CO oder gegebenenfalls mit einem bis drei Halogenresten substituiertes C₁₋₆-Alkyl-SO₂- steht;
sowie Salze, Isomere und Hydrate davon.

3. Verbindungen nach Anspruch 1,
worin
R¹ für einen Rest der Formel -O-SO₂-R³ steht,
wobei
R³ für einen Rest aus der Gruppe, bestehend aus Methyl, Ethyl, n-Propyl, iso-Propyl, n-Butyl, n-Pentyl, 1,1,1-Trifluor-4-n-butyl, Chlormethyl oder Cyclopropyl, steht;
R² für H oder CH₃CO steht;
sowie Salze, Isomere und Hydrate davon.

4. Verfahren zur Herstellung von Verbindungen der Formel 1, umfassend die Umsetzung der Verbindung der Formel (II) 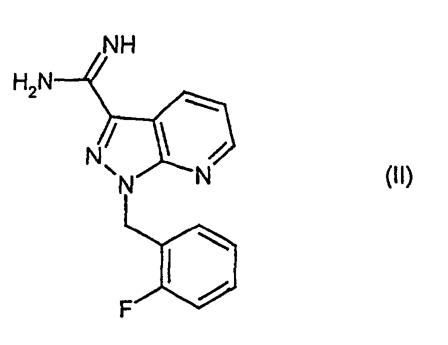 mit Verbindungen der Formel (III) 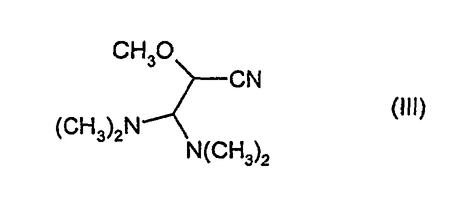 in einem organischen Lösungsmittel in Gegenwart einer Base unter Erhitzen und anschließender Überführung der Ethergruppe in die freie Hydroxygruppe zu Verbindungen der Formel (IV) 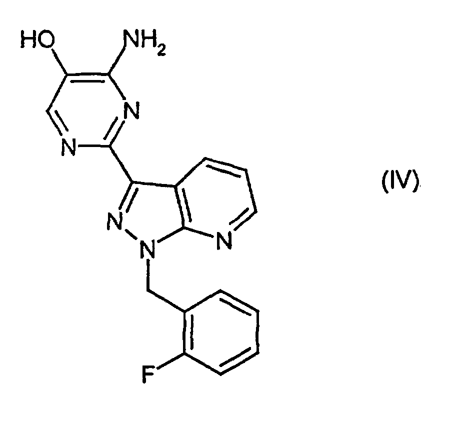 sowie die anschließende Umsetzung mit Verbindungen der Formel X-SO₂-R²
worin
X für eine durch eine Hydroxygruppe substituierbare Abgangsgruppe steht;
R² die in Anspruch 1 angegebene Bedeutung hat;
in einem organischen Lösungsmittel in Gegenwart einer Base unter Erhitzen zu Verbindungen der Formel (I).

5. Verbindungen der allgemeinen Formel (I) zur Behandlung von Krankheiten.

6. Arzneimittel enthaltend mindestens eine Verbindung der allgemeinen Formel (I) gemäß Anspruch 1.

7. Verfahren zur Herstellung von Arzneimitteln **dadurch gekennzeichnet, dass** man mindestens eine Verbindung der Formel (I) gemäß Anspruch 1, gegebenenfalls mit üblichen Hilfs- und Zusatzstoffen in eine geeignete Applikationsform überführt.

8. Arzneimittel enthaltend mindestens eine Verbindung der allgemeinen Formel (I) gemäß Anspruch 1 in Kombination mit organischen Nitraten oder NO-Donatoren.

9. Arzneimittel enthaltend mindestens eine Verbindung der allgemeinen Formel (I) gemäß Anspruch 1 in Kombination mit Verbindungen, die den Abbau von cyclischen Guanosinmonophosphat (cGMP) inhibieren.

10. Verwendung von Verbindungen der allgemeinen Formel (I) gemäß Anspruch 1 bei der Herstellung von Arzneimitteln zur Behandlung von Herz-Kreislauf-Erkrankungen.

11. Verwendung von Verbindungen der allgemeinen Formel (I) gemäß Anspruch 1 bei der Herstellung von Arzneimitteln zur Behandlung von Hypertonie.

12. Verwendung von Verbindungen der allgemeinen Formel (I) gemäß Anspruch 1 bei der Herstellung von Arzneimitteln zur Behandlung von thromboembolischen Erkrankungen und Ischämien.

13. Verwendung von Verbindungen der allgemeinen Formel (I) gemäß Anspruch 1 bei der Herstellung von Arzneimitteln zur Behandlung von sexueller Dysfunktion.

14. Verwendung von Verbindungen der allgemeinen Formel (I) gemäß Anspruch 1 bei der Herstellung von Arzneimitteln mit anitinflammatorischen Eigenschaften.

15. Verwendung von Verbindungen der allgemeinen Formel (I) gemäß Anspruch 1 bei der Herstellung von Arzneimitteln zur Behandlung von Erkrankungen des Zentralnervensystems.

16. Verwendung gemäß einem der Ansprüche 10 bis 15, wobei die Verbindungen der allgemeinen Formel gemäß Anspruch 1 in Kombination mit organischen Nitraten oder NO-Donatoren oder in Kombination mit Verbindungen, die den Abbau von cyclischen Guanosinmonophosphat (cGMP) inhibieren, eingesetzt werden.

## Claims

1. Compounds of the formula (I) 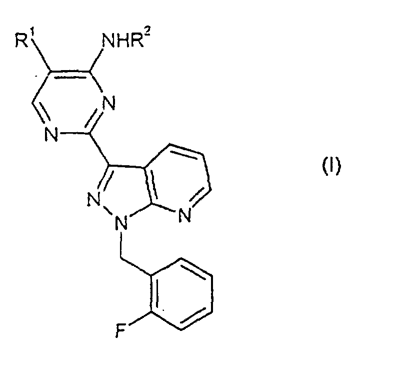 in which
R¹ is a radical of the formula -O-SO₂-R³,
where
R³ is a radical from the group consisting of optionally substituted C₁₋₆-alkyl, optionally substituted C₃₋₈-cycloalkyl, or optionally substituted phenyl;
R² is H, optionally substituted C₁₋₆-alkyl-CO or optionally substituted C₁₋₆-alkyl-SO₂-;
and salts, isomers and hydrates thereof.

2. Compounds according to Claim 1,
in which
R¹ is a radical of the formula -O-SO₂-R³,
where
R³ is a radical from the group consisting of C₁₋₆-alkyl which is optionally substituted by one to three halogen radicals, or C₃₋₈-cycloalkyl;
R² is H, C₁₋₆-alkyl-CO which is optionally substituted by one to three halogen radicals, or C₁₋₆-alkyl-SO₂- which is optionally substituted by one to three halogen radicals;
and salts, isomers and hydrates thereof.

3. Compounds according to Claim 1,
in which
R¹ is a radical of the formula -O-SO₂-R³,
where
R³ is a radical from the group consisting of methyl, ethyl, n-propyl, isopropyl, n-butyl, n-pentyl, 1,1,1-trifluoro-4-n-butyl, chloromethyl or cyclopropyl;
R² is H or CH₃CO;
and salts, isomers and hydrates thereof.

4. Process for preparing compounds of the formula (I), comprising the reaction of the compound of the formula (II) 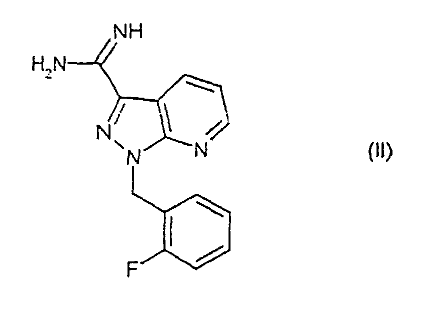 with compounds of the formula (III) 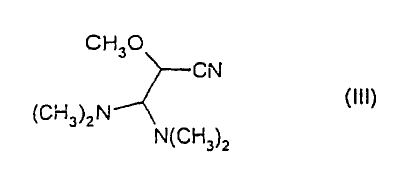 in an organic solvent in the presence of a base with heating and subsequent conversion of the ether group in the free hydroxyl group to give compounds of the formula (IV) 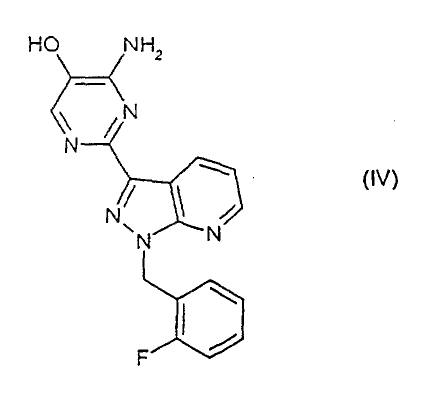 and subsequent reaction with compounds of the formula X-SO₂-R²
in which
X is a leaving group which can be replaced by a hydroxyl group;
R² has the meaning indicated in Claim 1;
in an organic solvent in the presence of a base with heating to give compounds of the formula (I).

5. Compounds of the formula (I) for treating diseases.

6. Medicament comprising at least one compound of the formula (I) according to Claim 1.

7. Process for producing medicaments, **characterized in that** at least one compound of the formula (I) according to Claim 1 is converted where appropriate with conventional excipients and additives into a suitable administration form.

8. Medicament comprising at least one compound of the formula (I) according to Claim 1 in combination with organic nitrates or NO donors.

9. Medicament comprising at least one compound of the formula (I) according to Claim 1 in combination with compounds which inhibit the breakdown of cyclic guanosine monophosphate (cGMP).

10. Use of compounds of the general formula (I) according to Claim 1 for the production of medicaments for the treatment of cardiovascular disorders.

11. Use of compounds of the general formula (I) according to Claim 1 for the production of medicaments for the treatment of hypertension.

12. Use of compounds of the general formula (I) according to Claim 1 for the production of medicaments for the treatment of thromboembolic disorders and ischaemias.

13. Use of compounds of the general formula (I) according to Claim 1 for the production of medicaments for the treatment of sexual dysfunction.

14. Use of compounds of the general formula (I) according to Claim 1 for the production of medicaments having anti-inflammatory properties.

15. Use of compounds of the general formula (I) according to Claim 1 for the production of medicaments for the treatment of disorders of the central nervous system.

16. Use according to any of Claims 10 to 15, where the compounds of the general formula according to Claim 1 are employed in combination with organic nitrates or NO donors or in combination with compounds which inhibit the breakdown of cyclic guanosine monophosphate (cGMP).

## Revendications

1. Composés de formule générale (I) 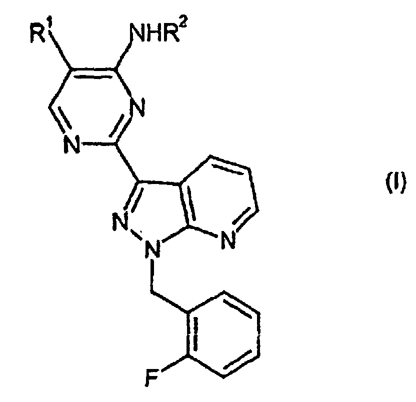 dans laquelle
R¹ représente un reste de formule -O-SO₂-R³,
où
R³ représente un reste du groupe constitué d'un reste alkyle en C₁ à C₆ éventuellement substitué, d'un reste cycloalkyle en C₃ à C₈ éventuellement substitué ou d'un reste phényle éventuellement substitué ;
R² représente H, un reste (alkyle en C₁ à C₆)-CO éventuellement substitué ou (alkyle en C₁ à C₆)-SO₂-éventuellement substitué ;
ainsi que leurs sels, leurs isomères et leurs hydrates.

2. Composés suivant la revendication 1,
dans lesquels
R¹ représente un reste de formule -O-SO₂-R³,
où
R³ est un reste du groupe constitué d'un reste alkyle en C₁ à C₆ qui est éventuellement substitué avec jusqu'à trois restes halogène, ou d'un groupe cycloalkyle en C₃ à C₈ ;
R² représente H, un reste (alkyle en C₁ à C₆)-CO éventuellement substitué avec jusqu'à trois restes
halogène, ou un reste (alkyle en C₁ à C₆)-SO₂-éventuellement substitué avec jusqu'à trois restes halogène ;
ainsi que leurs sels, leurs isomères et leurs hydrates.

3. Composés suivant la revendication 1,
dans lesquels
R¹ représente un reste de formule -O-SO₂-R³,
où
R³ est un reste du groupe constitué d'un reste méthyle, éthyle, n-propyle, isopropyle, n-butyle, n-pentyle, 1,1,1-trifluoro-4-n-butyle, chlorométhyle ou cyclopropyle ;
R² représente H ou CH₃CO ;
ainsi que leurs sels, leurs isomères et leurs hydrates.

4. Procédé de production de composés de formule (I), comprenant
la réaction à chaud du composé de formule (II) 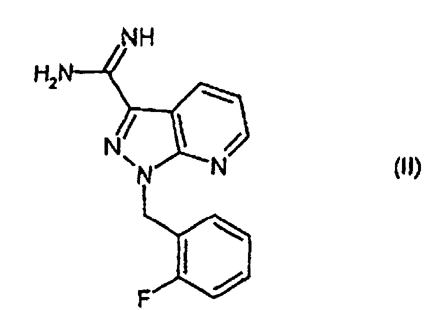 avec des composés de formule (III) 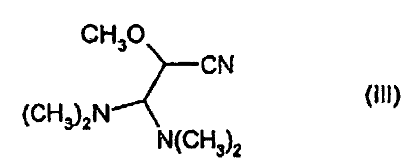 dans un solvant organique en présence d'une base, suivie de la transformation du groupe éther en groupe hydroxy libre pour obtenir des composés de formule (IV) 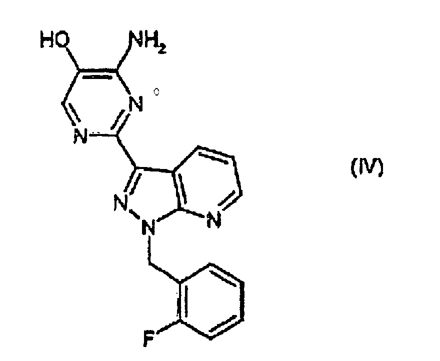 ainsi que la réaction subséquente avec des composés de formule X-SO₂-R²
dans laquelle
X est un groupe partant remplaçable par un groupe hydroxy ; R² a la définition indiquée dans la revendication 1 ; dans un solvant organique à chaud en présence d'une base pour obtenir des composés de formule (I).

5. Composés de formule générale (I), destinés au traitement de maladies.

6. Médicament contenant au moins un composé de formule générale (I) suivant la revendication 1.

7. Procédé de préparation de médicaments, **caractérisé en ce qu'**on fait prendre une forme d'administration convenable à au moins un composé de formule (I) suivant la revendication 1, éventuellement avec des agents auxiliaires et des additifs usuels.

8. Médicament contenant au moins un composé de formule générale (I) suivant la revendication 1, en association avec des nitrates organiques ou des donneurs de NO.

9. Médicament contenant au moins un composé de formule générale (I) suivant la revendication 1, en association avec des composés qui inhibent la dégradation du monophosphate de guanosine cyclique (GMPc).

10. Utilisation de composés de formule générale (I) suivant la revendication 1, pour la préparation de médicaments destinés au traitement de maladies du système cardiovasculaire.

11. Utilisation de composés de formule générale (I) suivant la revendication 1, pour la préparation de médicaments destinés au traitement de l'hypertonie.

12. Utilisation de composés de formule générale (I) suivant la revendication 1, pour la préparation de médicaments destinés au traitement de maladies thromboemboliques et d'ischémies.

13. Utilisation de composés de formule générale (I) suivant la revendication 1, pour la préparation de médicaments destinés au traitement d'un dysfonctionnement sexuel.

14. Utilisation de composés de formule générale (I) suivant la revendication 1, pour la préparation de médicaments doués de propriétés anti-inflammatoires.

15. Utilisation de composés de formule générale (I) suivant la revendication 1, pour la préparation de médicaments destinés au traitement de maladies du système nerveux central.

16. Utilisation suivant l'une des revendications 10 à 15, dans laquelle les composés de formule générale suivant la revendication 1 sont utilisés en association avec des nitrates organiques ou des donneurs de NO ou en association avec des composés qui inhibent la dégradation du monophosphate de guanosine cyclique (GMPc).
